(19) Europäisches Patentamt / European Patent Office / Office européen des brevets

(11) **EP 0 880 536 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**05.08.2015 Patentblatt 2015/32**

(45) Hinweis auf die Patenterteilung:
**23.04.2003 Patentblatt 2003/17**

(21) Anmeldenummer: **97901080.8**

(22) Anmeldetag: **24.01.1997**

(51) Int Cl.:
*C07H 1/08* (2006.01)    *A61K 48/00* (2006.01)
*C12N 15/10* (2006.01)

(86) Internationale Anmeldenummer:
**PCT/EP1997/000321**

(87) Internationale Veröffentlichungsnummer:
**WO 1997/029113 (14.08.1997 Gazette 1997/35)**

(54) **VERFAHREN ZUR HERSTELLUNG VON GEREINIGTER NUKLEINSÄURE UND DEREN VERWENDUNG**

PROCESS FOR PREPARING PURIFIED NUCLEIC ACID AND THE USE THEREOF

PROCEDE POUR LA PREPARATION D'ACIDE NUCLEIQUE PURIFIE ET UTILISATION DE CE DERNIER

(84) Benannte Vertragsstaaten:
**AT BE CH DE DK ES FR GB IE IT LI NL**

(30) Priorität: **06.02.1996 EP 96101628**

(43) Veröffentlichungstag der Anmeldung:
**02.12.1998 Patentblatt 1998/49**

(73) Patentinhaber: **Roche Diagnostics GmbH**
**68305 Mannheim (DE)**

(72) Erfinder: **KUHNE, Wolfgang**
**D-82377 Penzberg (DE)**

(74) Vertreter: **Vossius & Partner**
**Patentanwälte Rechtsanwälte mbB**
**Siebertstrasse 3**
**81675 München (DE)**

(56) Entgegenhaltungen:
EP-A- 0 268 946    WO-A-95/21177
WO-A-95/21179    WO-A-95/21250

- N. A. HORN ET AL.: 'Cancer Gene Therapy Using Plasmid DNA: Purification of DNA for Human Clinical Trials' HUMAN GENE THERAPY Bd. 6, Mai 1995, Seiten 565 - 573
- M. COTTEN ET AL.: 'Lipopolysaccharide is a frequent contaminant of plasmid DNA preparations and canbe toxic to primary human cells in the presence of adenovirus' GENE THERAPY Bd. 1, 1994, Seiten 239 - 246
- T. R. JOHNSON ET AL.: 'Large-Scale Isolation of Plasmid DNA and Purification of Phage DNA Using Hydroxylapatite Chromatography' ANALYTICAL BIOCHEMISTRY Bd. 132, 1983, Seiten 20 - 25
- P. M. MONTBRIAND ET AL.: 'Improved method for the removal of endotoxin from DNA' JOURNAL OF BIOTECHNOLOGY Bd. 44, 1996, Seiten 43 - 46
- M. MARQUET ET AL.: 'Process Development for the Manufacture of Plasmid DNA Vectors for Use in Gene Therapy' BIOPHARM September 1995, Seiten 26 - 37
- European Pharmacopoeia 2004, 2.6.14 Bacterial Endotoxins
- 'The United States Pharmacopeia', 01 Januar 2004 Seiten 2169 - 2173
- Versuchsbericht Philip Henwood
- Stellungnahme Dr. Kuhne
- 'Qiagen Plasmid Purification Handbook', 1999 Seiten 64 - 66
- 'Qiagen Plasmid Purification Handbook', 2012 Seiten 36 - 39

EP 0 880 536 B2

**Beschreibung**

Die Erfindung betrifft die Herstellung von gereinigter Nukleinsäure und deren Verwendung, insbesondere in der Gentherapie.

Die Herstellung von replizierbarer Nukleinsäure erfolgt üblicherweise durch Vermehrung von replikationsfähiger Plasmid-DNA in gramnegativen Bakterien, wie z.B. E. coli. Nach Aufschluß der Biomasse (üblicherweise alkalische Lyse, mit Lysozym oder Ultraschall) wird abzentrifugiert und der Überstand mit Phenol ausgeschüttelt. Anschließend wird an einem Cäsiumchloridgradienten eine Ultrazentrifugation durchgeführt. (Birnboim & Doly, Nucleic Acid Res. 7 (1979) 1513 - 1523, Garger et al., Biochem. Biophys. Res. Comm. 117 (1983) 835 - 842.). Derartige Präparationen enthalten jedoch Endotoxine, Phenol, Cäsiumchlorid und/oder Ethidiumbromid als Anfärbemittel.

Ein weiteres Verfahren ist im QIAGEN® Plasmid Handbook (Qiagen Inc., Chatsworth, USA) und der EP-B 0 268 946 beschrieben. Danach wird das nach üblichem Aufschluß gewonnene Zellysat an QIAGEN®-TIP, welches QIAGEN® resin (ein Trägermaterial auf Silicagelbasis) enthält, chromatographiert. Der Nachteil dieses Verfahrens ist, daß DNA-Bindeproteine nicht vollständig von der DNA abgelöst werden und deshalb die gereinigte Plasmidfraktion Proteine und insbesondere Endotoxine (aus der Membran der gramnegativen Wirtszellen) in beträchtlichem Umfang enthält.

In einem weiteren Verfahren wird nach alkalischer Lyse der E.coli-Biomasse nach Birnboim & Doly eine Chromatographie des Zentrifugationsüberstandes unter Hochsalzbedingungen über Anionentauschersäulen (z. B. Mono-Q, Source-Q von Pharmacia, Macroprep-Q von BioRad, Poros-Q von Perseptive Biosystems oder HyperD-Q von Biosepra, vgl. Chandra et al., Analyt. Biochem. 203 (1992) 169 - 172; Dion et al., J. Chrom. 535 (1990) 127 - 147) durchgeführt. Auch hier enthält die gereinigte Plasmidfraktion Proteine und insbesondere in beträchtlichem Umfang Endotoxine.

In einem weiteren Verfahren ist nach alkalischer Lyse und anschließender Phenol/Chloroformextraktion eine Chromatographie über Gelfiltration möglich (McClung & Gonzales, Analyt. Biochem. 177 (1989) 378-382; Raymond et al., Analyt. Biochem. 173 (1988) 125 - 133). Auch nach dieser Reinigung enthält die Plasmidpräparation Verunreinigungen, insbesondere Phenol.

In WO 95/21177 und WO 95/21179 wird ein Verfahren zur Isolierung und Reinigung von Nukleinsäuren für den Einsatz in der Gentherapie beschrieben, wobei die Reinigung im wesentlichen durch Zentrifugieren, Filtrieren, eine Affinitätschromatographie oder eine Chromatographie an einem anorganischen Chromatographiematerial, mit anschließender Chromatographie an einem Ionenaustauscher, erfolgt. Eine weitere Abreicherung von Endotoxinen kann nach der WO 95/21177 dann erreicht werden, wenn im Reinigungprozeß die Nukleinsäure mit einem "Endotoxin Removal Buffer", der 10 % Triton® X 100 und 40 mmol/l MOPS-Puffer (3-Morpholino-1-propansulfonat-puffer) bzw. 50 mmol/l MOPS-Puffer (WO 95/21179) enthält, behandelt wird. Ein Nachteil dieses Verfahrens besteht darin, daß die auf diese Weise gereinigte Nukleinsäure Verunreinigungen an Triton® und MOPS-Puffer enthält. Durch dieses Verfahren kann zwar eine Abreicherung von Endotoxinen auf einen Gehalt von ca. 100 EU/mg DNA (Qiagen News 1/96, 3 - 5) bzw. gemäß WO 95/21179 von 10 - 17 EU/mg DNA erreicht werden, eine weitergehende Entfernung von Endotoxinen ist jedoch durch dieses Verfahren nicht möglich.

Für eine therapeutische Anwendung, wie sie beispielsweise für die Gentherapie vorgesehen ist, wird jedoch eine Nukleinsäurepräparation benötigt, die möglichst frei von allen Verunreinigungen (insbesondere weitestgehend frei von Endotoxinen) ist. Vor allem der Endotoxingehalt von Plasmidpräparationen stellt bisher ein nicht gelöstes Problem dar, wie beispielsweise von Cotten et al., Gene Therapy 1 (1994) 239 - 246 beschrieben wurde. Ein verminderter Endotoxingehalt (ca. 100 EU/mg DNA) kann nach dem Stand der Technik, wie beispielsweise gemäß der WO 95/21177, nur dann erreicht werden, wenn die Nukleinsäuren mit nichtionischen Detergentien, wie z. B. Triton (Endotoxin Removal Buffer aus WO 95/21177), behandelt werden. Triton® hat jedoch eine biologische Wirkung, wie z. B. Lungenveränderungen oder Senkung des Blutdrucks (Fiedler, Lexikon der Hilfsstoffe für Pharmazie und Kosmetik und angrenzende Gebiete (Band 9, 3. Auflage, 1989, Editio Cantor, DE)). Der zusätzlich erforderliche MOPS-Puffer enthält ebenfalls, aus Sicht einer therapeutischen Anwendung, eine problematische Substanz.

Durch die Erfindung wird eine Präparation, einer Plasmid-DNA von hoher Reinheit, in der die Endotoxine weitgehend abgereichert sind, vorzugsweise ohne Ethidiumbromid, Phenol, Cäsiumchlorid, Polymyxin oder nichtionischen Detergentien sowie ein einfaches und effektives Verfahren zur Reinigung von solchen Nukleinsäuren, insbesondere zur Abreicherung von Endotoxinen bereitgestellt.

Gegenstand der Erfindung ist eine in gramnegativen Bakterien replizierbare Plasmid-DNA mit einem Gehalt von weniger als 0,1 % Protein und einem Gehalt von 0,01 - 0,1 EU/mg DNA an Endotoxinen. Vorzugsweise ist diese Plasmid-DNA frei von Ethidiumbromid, Phenol und Cäsiumchlorid, frei von Detergenzien auf Basis von Octylphenolpoly(ethylenglycolether)$_n$, wie z.B. Triton®-Detergenzien, ebenso frei von MOPS-Puffersubstanz und von RNAse.

Unter Amplifikation ist die Erhöhung der Kopienzahl einer Nukleinsäure (insbesondere DNA und Plasmid-DNA), basierend auf der Replikation eines Vektors zu verstehen. Dabei werden von einem template eine Vielzahl von Kopien hergestellt. Repliziert wird ein Vektor, welcher die Nukleinsäure darstellt, oder welcher die Nukleinsäure kloniert enthält.

Unter einer Plasmid-DNA ist ein extrachromosomales DNA-Duplex-Molekül zu verstehen. Die Größe eines DNA-Plasmids beträgt üblicherweise 1 bis mehr als 200 kb und liegt in Wirtszellen in einer bis mehreren Hunderten von Kopien vor. Plasmid-DNA wird üblicherweise in gramnegativen Bakterien, wie z. B. E.coli, amplifiziert und anschließend isoliert. Plasmide werden häufig verwendet zur Konstruktion von Klonierungsvektoren, zur Transfektion von prokaryontischen und eukaryontischen Zellen. Eine therapeutische Verwendung ist insbesondere im Zusammenhang mit der in vivo- und ex vivo-Gentherapie von Bedeutung. Therapeutisch verwendete Plasmid-DNA hat vorzugsweise eine Länge von 5 bis 20 kb, besonders bevorzugt 5 - 10 kb, und ist doppelsträngig. Die Plasmid-DNA kann linearisiert oder zirkulär geschlossen sein. Vorzugsweise wird im wesentlichen zirkulär geschlossene DNA verwendet.

Ein weiterer Gegenstand der Erfindung ist demzufolge eine pharmazeutische Zusammensetzung, enthaltend eine erfindungsgemäße Plasmid-DNA, in einer therapeutisch wirksamen Menge und gegebenenfalls zusätzlich pharmazeutische Hilfs-, Füll oder Zusatzstoffe.

Endotoxine sind Lipopolysaccharide aus gramnegativen Bakterien. Endotoxine können in Säugern als Pyrogene wirken und einen Endotoxinschock induzieren. Die wesentliche toxische Komponente der Endotoxine ist Lipid A, wobei der Polysaccharidteil die Wasserlöslichkeit vermittelt und der Lipidteil toxisch wirkt. Der biologische Effekt von Endotoxinen in Säugern sind insbesondere eine Hypersensibilisierung sowie andere Reaktionen, die durch Fieber begleitet sind.

Plasmid-DNA wird gemäß den Standardverfahren in E.coli, also einem gramnegativen Bakterium, amplifiziert. Nach der Fermentation wird die dabei erhaltene Biomasse aufgeschlossen und die Zellen lysiert. Hierbei werden aus der Zellmembran die Endotoxine freigesetzt. Dies bedeutet, daß nach der Amplifikation von erfindungsgemäß hergestellten Nukleinsäuren, insbesondere von Plasmid-DNA, in gramnegativen Bakterien und insbesondere in E.coli eine Abreicherung von Endotoxinen erforderlich ist, wenn diese Plasmid-DNA therapeutisch verwendet werden soll.

Für eine therapeutische Anwendung von erfindungsgemäß hergestellten replizierbaren Nukleinsäuren, insbesondere Plasmid-DNA, werden, je nach Anwendung, Dosen von 50 $\mu$g bis 10 mg und mehr verwendet bzw. geplant. Die Dosismenge hängt von der Erkrankung und Applikationsart ab. Im Aerosol, z. B. zur Behandlung der cystischen Fibrose, werden Dosen von 400 $\mu$g und mehr verwendet. Analoges. gilt für im Lipidkomplex (z. B. in Liposomen) verkapselte Plasmid-DNA. Um derartige Mengen von therapeutisch einsetzbarer replizierbarer Nukleinsäure zur Verfügung zu stellen, ist es erforderlich, die replizierbare Nukleinsäure in großem Maßstab herzustellen. Hierfür sind Fermentationsansätze mit 1 - 5 kg Biomasse, woraus 1 - 5 g Nukleinsäure isoliert werden können, zweckmäßig.

Gegenstand der Erfindung ist ebenfalls ein Verfahren zur Herstellung einer Nukleinsäure, vorzugsweise einer Plasmid-DNA mit einem Gehalt von weniger als 1 EU/mg DNA, bevorzugt 0,01 - 0,1 EU/mg DNA an Endotoxinen, welches dadurch gekennzeichnet ist, daß Plasmid-DNA in gramnegativen Bakterien wie E.coli vermehrt wird, die Biomasse aufgeschlossen und die löslichen Bestandteile an Hydroxylapatit chromatographiert werden und anschließend die genannte Plasmid-DNA isoliert wird. Es wird vor der Chromatographie an Hydroxylapatit eine Anionentauscherchromatographie durchgeführt, mit der im wesentlichen RNA und Fremdproteine entfernt werden. Dadurch können gegebenenfalls weitere Verunreinigungen beseitigt werden und ein Gehalt der Nukleinsäure von weniger als 1 % Protein, vorzugsweise weniger als 0,1 % Protein, frei von Ethidiumbromid, Phenol und Cäsiumchlorid erreicht werden. Eine solche Präparation ist vorzugsweise auch frei von Detergentien auf Basis von Octylphenolpoly(ethylenglycolether)$_n$ und MOPS-Puffersubstanz.

Durch das erfindungsgemäße Verfahren gelingt es, eine Vielzahl von Verunreinigungen zu vermeiden oder zu beseitigen, die Plasmid-DNA enthält, wenn sie nach einem dem Fachmann geläufigen Verfahren hergestellt wird. Vor allem gelingt es überraschenderweise auf einfache Weise, den Endotoxingehalt drastisch zu vermindern.

Im erfindungsgemäßen Verfahren wird durch die Chromatographie mit Hydroxylapatit eine überragende Abreicherung von Endotoxinen erreicht. Dies ist um so überraschender, da die Chromatographie an Hydroxylapatit in der Literatur lediglich zur Trennung von DNA und RNA eingesetzt wird (Johnson & Ilan, Analyt. Biochem. 132 (1983) 20 - 25).

Die chromatographische Wirkung von Hydroxylapatit beruht im wesentlichen auf der Wechselwirkung zwischen Calcium$^{2+}$-Gruppen und der negativen Ladung der zu reinigenden Nukleinsäure und in geringerem Umfang durch die Wechselwirkung der zu reinigenden Nukleinsäure mit PO$_4{}^{3-}$-Gruppen an der Oberfläche von kristallinem Hydroxylapatit (vgl. z. B. Protein Purification Methods, Ed. by Elv. Harries and S. Angal, Oxford University Press 1989, 238 - 244). Im wesentlichen kann die Chromatographie an Hydroxylapatit für Nukleinsäuren als Anionenaustauscherschritt bezeichnet werden, wobei die gebundene DNA nicht durch einfache Erhöhung der Ionenstärke (z. B. NaCl) sondern durch Erhöhung der Konzentration, vorzugsweise an Phosphat oder Citrat, zweiwertigen Metallionen und/oder EDTA, von der Hydroxylapatit-Matrix eluiert werden kann.

Im erfindungsgemäßen Verfahren werden bei der Chromatographie an Hydroxylapatit (z. B. HA-Ceramic, Bio-Gel HPHT, Bio-Gel HT/HTP von Biorad DE, HA-Ultrogel von IBF oder HA spheroidal von BDH, Macrosorb C von Sterling Organics) zunächst Endotoxine und die zu reinigende Nukleinsäure im wesentlichen über Dipol-Dipol Wechselwirkungen an Hydroxylapatit gebunden. Die Äquilibrierung erfolgt üblicherweise bei neutralem pH in Phosphatpuffer. Vorzugsweise wird, wie beim anschließenden Waschen der Säule, ein Denaturierungsmittel zugegeben. Überraschenderweise gelingt es mit Phosphat-, Citrat- oder Calciumionen, die Nukleinsäure aus ihrer Bindung an Hydroxylapatit zu verdrängen, während die Endotoxine gebunden bleiben. Die Verdrängung der Nukleinsäure aus ihrer Bindung an Hydroxylapatit kann, statt durch Calciumionen, auch durch andere zweiwertige Metallionen, die in Apatit Calcium ersetzen können, wie z. B. Mg, Fe, Mn, durchgeführt werden. Zur Elution beträgt die Ionenkonzentration vorzugsweise 100 mmol/l oder mehr. Besonders bevorzugt sind Ionenkonzentrationen zwischen 100 und 500 mmol/l bzw. 200 und 500 mmol/l. Besonders bevorzugt wird eine phosphationenhaltige Lösung (z. B. Phosphatpuffer) verwendet. Vor der Elution (ohne Denaturierungsmittel) wird zweckmäßig (mit Denaturierungsmittel) gewaschen. Es hat sich als vorteilhaft gezeigt, wenn z. B. eine Phosphat- oder Sulfatlosung (100 - 200 mmol/l) verwendet wird, der ein Denaturierungsmittel (z. B. Harnstoff oder Guanidinhydrochlorid) in einer DNA-denaturierenden Konzentration (z. B. 6 mol/l Harnstoff) zugegeben wird.

In einer bevorzugten Ausführungsform wird zusätzlich noch eine Ultrafiltration nach der Chromatographie an Hydroxylapatit durchgeführt.

Zur erfindungsgemäßen Herstellung der Nukleinsäure werden die Plasmide, welche die Nukleinsäure darstellen oder enthalten, üblicherweise in gramnegativen Bakterienkulturen amplifiziert. Derartige Methoden sind dem Fachmann bekannt und beispielsweise von J. Sambrook et al. (1989), Molecular Cloning: A Laboratory Manual, 2nd edition, Cold Spring Harbor Laboratory Press und von F.M. Ausubel et al. eds. (1991), Current Protocols in Molecular Biology, Wiley Interscience, New York beschrieben. Dazu werden die Bakterienkulturen, welche die Plasmide enthalten, zunächst subkultiviert und anschließend in einem geeigneten Medium, gegebenenfalls unter Zusatz eines Selektionsmittels kultiviert.

Der Aufschluß der Biomasse erfolgt ebenfalls nach dem Fachmann geläufigen Methoden (mechanischer oder enzymatischer Aufschluß, siehe z.B. Birnboim und Doly, Nucleic Acids Research 7 (1979) 1513-1523), ohne RNAse-Zugabe. Auf eine Phenolausschüttelung kann verzichtet werden, wenn die Proteinabtrennung durch die Chromatographie an einem Anionentauscher erfolgt. Nach Aufschluß und Abtrennung der unlöslichen Bestandteile, zweckmäßig durch Zentrifugation und Filtration über eine Filterkerze (5 $\mu$m Poren), wird der Zellüberstand zur Entfernung von Proteinen zunächst an einem Anionentauscher chromatographiert. Als Anionentauscher geeignet sind Anionentauscher auf Agarosebasis, wie beispielsweise Q-Sepharose. Weitere geeignete Anionenaustauscher basieren auf Polymetacrylat (Macroprep/Bio-Rad, Deutschland), Polystyrol/Divinylbenzol (Poros/Perseptive, HyperD/Biosepra, Source/Pharmacia) oder Silicagel, an dessen Oberfläche z. B. Diethylaminoethyl (DEAE) oder Dimethylaminoethyl(DMAE)-gruppen gebunden sind.

Um den Reinigungseffekt zu optimieren, wird die Nukleinsäure mittels Hochsalzgradient, z. B. NaCl-Gradient (vorzugsweise 0,65 mol/l - 0,85 mol/l) in TE-Puffer eluiert. Dadurch wird überraschenderweise eine Abtrennung einer Vielzahl von Verunreinigungen (RNA, Protein) in einem Schritt möglich.

Ebenso bevorzugt ist es, noch zusätzlich, vorzugsweise nach der Hydroxylapatitchromatographie, eine Isopropanol-/Ethanolfällung zur Minimierung der Keimbelastung sowie zur Entsalzung durchzuführen. Anschließend kann die erfindungsgemäß hergestellte Nukleinsäure steril abgefüllt werden.

Die nachfolgenden Beispiele beschreiben die Erfindung weiter:

**Beispiel 1**

**Gewinnung von Nukleinsäure aus E.coli-Biomasse**

Plasmid-DNA enthaltende E.coli-Biomasse wird durch eine Alkali-Lyse aufgeschlossen und die freigesetzte Plasmid-DNA über Q-Sepharose und HA-Ceramic chromatographiert. Das Eluat wird durch eine Isopropanol/Ethanol-Fällung entsalzt und konzentriert und der Plasmid-DNA-Niederschlag in TE-Puffer resuspendiert.

Resuspensionspuffer: 50 mmol/l Tris/HCl, 10 mmol/EDTA-Na$_2$, pH 8,0 $\pm$ 0,2

Kaliumacetatpuffer: 3 mol/l Kaliumacetatpuffer pH 5,5

60 g Biomasse (feucht, E.coli) wird aus dem Fermenter in entpyrogenisierte Zentrifugenbecher abgefüllt. Es werden 750 ml Resuspensionspuffer zugegeben und mindestens 24 Stunden bei 5±4°C langsam (ca. 35 RPM) gerührt, bis die Biomasse vollständig suspendiert ist. Dabei wird die Temperatur der Suspension langsam auf 25°C erhöht.

Zur Suspension werden 750 ml 0,2 mol/l NaOH/1 % SDS unter Rühren mit ca. 80 RPM zugegeben und bei 25°C 5 Minuten inkubiert. Es werden 750 ml Kaliumacetatpuffer (s. o.) unter Rühren zugefügt und die Temperatur der Biomasse möglichst schnell auf 4°C abgesenkt.

Die Biomasse wird für 30 Minuten bei 26000 xg und 4°C zentrifugiert. Der Überstand, der die Plasmid-DNA enthält, wird dekantiert und über eine 5 $\mu$m-Filterkerze klarfiltriert.

**Chromatographie an Q-Sepharose ff:**

TE-Puffer: 10 mmol/l Tris-HCl, 1 mmol/l EDTA pH 8,0 $\pm$ 0,2

Äquilibrier/Waschpuffer = Gradientenpuffer A: 10 mmol/l Tris-HCl, 1 mmol/l EDTA, 0,65 mol/l NaCl pH 8,0 $\pm$ 2.

Gradientenpuffer B: 10 mmol/l Tris-HCl, 1 mmol/l EDTA, 0,85 mol/l NaCl pH 8,0 $\pm$ 0,2.

Der dekantierte Zentrifugenüberstand wird durch Zugabe von ca. 350 ml TE-Puffer/l Zentrifugationsüberstand auf 49 - 50 mS/cm Leitfähigkeit eingestellt und auf 5° $\pm$ 4°C abgekühlt. Die gesamte Chromatographie wird bei dieser Temperatur durchgeführt. Der Zentrifugationsüberstand wird bei 5 - 8 Säulenvolumen (SV)/Std. auf die äquilibrierte Säule aufgezogen. Anschließend wird die Säule bei einer Flußrate von 5 - 8 SV/Std. mit ca. 8 SV 10 mmol/l Tris-HCl, 1 mmol/l EDTA, 0,65 mol/l NaCl pH 8,0 ± 0,2 gewaschen.

**Elution**

An die Säule wird ein Gradient (5 SV Puffer A, 5 SV Puffer B) angelegt und das Eluat bei einer Flußrate von 5 - 8 SV/Std. fraktioniert. Die Detektion erfolgt bei 254 nm. Der Vorpeak (Verunreinigungen) wird vom Hauptpeak (Plasmid-DNA) abgetrennt, indem der Hauptpeak ab ansteigender Flanke in einem separaten Gefäß aufgefangen wird. Der Endotoxingehalt des Eluats beträgt zwischen 1200 und 12000 EU/mg Plasmid-DNA.

**Chromatographie an Hydroxylapatit (HA Ceramic)**

Die Chromatographie wird bei 5 ± 4°C durchgeführt.

Äquilibrierungspuffer: 0,1 mol/l Kaliumphosphat, 6 mol/l Harnstoff pH 7,0 ± 0,2

Waschpuffer 1: 0,15 mol/l Kaliumphosphat, 6 mol/l Harnstoff pH 7,0 ± 0,2

Waschpuffer 2: 0,02 mol/l Kaliumphosphat-Puffer pH 7,0 ± 0,2

Elutionspuffer: 0,5 mol/l Kaliumphosphat pH 7,0 ± 0,2

Die Detektion erfolgt bei 254 nm mit einer UV-Detektor/Schreibereinheit. Als Eichlösung wird eine 1 % Produktlösung (Plasmid DNA), vermessen mit einem kalibrierten Photometer, verwendet.

Der Q-Sepharose®-Pool wird auf eine Endkonzentration von 1,1 mmol/l Calciumchlorid gebracht und bei einer Flußrate von 5 - 8 SV/Std. auf die äquilibrierte Säule aufgezogen.

Anschließend wird die Säule bei einer Flußrate von 5 - 8 SV/Std. nacheinander gewaschen mit:

1. 0,1 mol/l Kaliumphosphat, 6 mol/l Harnstoff pH 7,0 ± 0,2, bis am Detektor keine Absorption mehr erkennbar ist.
2. 2 - 4 SV, 0,15 mol/l Kaliumphosphat, 6 mol/l Harnstoff pH 7,0 ± 0,2
3. 5 SV, 0,02 mol/l Kaliumphosphat pH 7,0 ± 0,2

Im Anschluß an die Waschschritte wird mit 0,5 mol/l Kaliumphosphatpuffer pH 7,0 ± 0,1 bei einer Flußrate von 5 - 6 SV/Std. eluiert.

Der Peak wird gesammelt, auf 25°C temperiert und mit 10 % seines Volumens an 4 mol/l KCl-Lösung aufgestockt. Anschließend wird mit 0,7 Vol.-Anteil (bezogen auf das Volumen des Eluats) an Isopropanol versetzt, die Losungen gemischt und für 5 - 10 Minuten bei 25°C inkubiert. Es wird für 30 Minuten bei ≥20000 xg zentrifugiert, wobei sich die Plasmid-DNA im Niederschlag befindet.

Der Niederschlag wird mit 20 ml 70 %igem Ethanol versetzt und für 10 - 15 Minuten bei ≥20000 xg bei 4°C erneut abzentrifugiert.

Der Niederschlag, welcher die Plasmid-DNA enthält, wird in TE-Puffer (10 mmol/l Tris-HCl, 1 mmol/l EDTA pH 8,0 ± 0,2) resuspendiert und eine Plasmidkonzentration von 1 mg/ml wird eingestellt. Der Endotoxingehalt liegt typischerweise bei weniger als 0,06 EU/mg DNA und zwischen 0,01 und 0,06 EU/mg DNA

Die Bestimmung des Endotoxingehalts erfolgt durch Zugabe der zu untersuchenden Lösung zu einer Limulus-Amöbozyten-Lysat-Lösung (LAL-Lösung). Endotoxine führen zu einer Gelbildung in diesem Gemisch.

In den Negativkontrollansätzen darf es zu keiner Gelbildung kommen, und in den Positivkontrollansätzen sowie in den Probelösungen, aufgestockt mit zwei λ-Kontrollstandard-Endotoxin, muß es zu einer Gelbildung kommen.

Die erste Verdünnungsstufe der Wirkstofflösung, für die diese Kriterien zutreffen und bei der es zu keiner Gelbildung kommt, wird zur Berechnung des Endotoxingehalts der Wirkstofflösung nachfolgende Formel eingesetzt:

$$E = V \times \lambda \ (EE/ml)$$

E: Endotoxingehalt
V: Verdünnungsfaktor
λ: Lysatempfindlichkeit (EE/ml)

**Beispiel 2**

**Plasmidpräparation nach dem Stand der Technik**

Die Plasmidpräparation erfolgt analog Birnboim et al., Nucl. Acids Res. 7 (1979) 1513-1523 und Meth. Enzymol. 100 (1983) 243-255. Danach werden die Bakterienzellen in NaOH/SDS, in Gegenwart von RNase, lysiert. Es wird zentrifugiert und der Überstand, welcher die Plasmid-DNA enthält, weiterverarbeitet. Der Überstand wird auf eine voräquilibrierte Qiagen®-Säule geladen.

Die Bakterienmasse wird in 4 ml Puffer (100 μg/ml RNase A, 50 mmol/l Tris-HCl, 10 mmol/l EDTA, pH 8,0) resuspendiert. Es werden 4 ml Lysepuffer (200 mmol/l NaOH, 1 % SDS zugegeben und bei Raumtemperatur für 5 Minuten inkubiert. Anschließend werden 4 ml Neutralisationspuffer (3 mol/l Kaliumacetat, pH 5,5) zugegeben und bei 4°C für 15 Minuten inkubiert. Bei der gleichen Temperatur wird 30 Minuten bei 30000 xg zentrifugiert und der Überstand weiterverarbeitet. Eine Qiagen®-Säule wird mit 4 ml Äquilibrierungspuffer (750 mmol/l NaCl, 50 mmol/l MOPS, 15 % Ethanol, pH 7,0, 0,15 % Triton®X 100) äquilibriert und der Überstand auf die Säule gegeben. Es wird mit 1 mol/l NaCl, 50 mmol/l MOPS, 15 % Ethanol, pH 7,0 gewaschen und mit 5 ml Elutionspuffer (1,25 mol/l NaCl, 50 mmol/l Tris-HCl, 15 % Ethanol, pH 8,5) eluiert.

Das Eluat wird mit Isopropanol (0,7 Vol.) präzipitiert und bei 15000 xg bei 4°C für 30 Minuten zentrifügiert. Das DNA-Pellet wird in 70 % Ethanol gewaschen und nochmals zentrifugiert. Anschließend wird das Pellet in 10 mmol/l Tris-HCl, 1 mmol/l EDTA, pH 8,0 resolubilisiert.

Der Endotoxingehalt einer solchen Plasmidpräparation beträgt typischerweise 300 - 3000 EU/mg. Unter Verwendung eines "Endotoxin Removal Buffers" gemäß WO 95/21177 und Qiagen News 1/96, S. 3 - 5 kann der Endotoxingehalt noch auf ca. 100 EU/mg gesenkt werden.

**Referenzliste**

Ausubel, F.M., et al. eds. (1991), Current Protocols in Molecular Biology, Wiley Interscience, New York

Birnboim, H.C. und Doly, J., Nucleic Acids Research 7 (1979) 1513-1523

Birnboim, H.C., et al., Meth. Enzymol. 100 (1983) 243-255

Chandra et al., Analyt. Biochem. 203 (1992) 169-172

Cotten et al., Gene Therapy 1 (1994) 239-246

Dion et al., J. Chrom. 535 (1990) 127-147

Europäisches Patent EP-B 0 268 946

Fiedler, Lexikon der Hilfsstoffe für Pharmazie und Kosmetik und angrenzende Gebiete (Band 9, 3. Auflage, 1989, Editio Cantor, DE)

Garger et al., Biochem. Biophys. Res. Comm. 117 (1983) 835-842

Johnson & Ilan, Analyt. Biochem. 132 (1983) 20-25

McClung & Gonzales, Analyt. Biochem. 177 (1989) 378-382

Protein Purification Methods, Ed. by Elv. Harries and S. Angal, Oxford University Press 1989, 238-244.

QIAGEN NEWS 1/96, 3-5

QIAGEN Plasmid Handbook (Qiagen Inc,. Chatsworth, USA)

Raymond et al., Analyt. Biochem. 173 (1988) 125-133

Sambrook, J., et al. (1989), Molecular Cloning: A Laboratory Manual, 2nd edition, Cold Spring Harbor Laboratory Press, New York, USA

WO 95/21177

**Patentansprüche**

1. Präparation einer in gramnegativen Bakterien replikationsfähigen Plasmid-DNA mit einem Gehalt von weniger als 0,1 % Protein sowie mit einem Gehalt von 0,01 bis 0,1 EU/mg DNA an Endotoxinen.

2. Präparation nach Anspruch 1, frei von Ethidiumbromid, Phenol, Cäsiumchlorid, MOPS-Puffersubstanz und Detergenzien auf Basis von Octylphenolpoly(ethylenglycolether)$_n$.

3. Pharmazeutische Zusammensetzung, enthaltend eine replizierbare Plasmid-DNA mit einem Gehalt von weniger als 0,1 % Protein sowie mit einem Gehalt von 0,01 bis 0,1 EU/mg DNA an Endotoxinen in einer therapeutisch wirksamen Menge und gegebenenfalls pharmazeutische Hilfs-, Füll- oder Zusatzstoffe.

4. Verfahren zur Herstellung einer Nukleinsäure mit einem Gehalt von weniger als 1 EU/mg DNA an Endotoxinen, **dadurch gekennzeichnet, dass** ein Plasmid, welches die Nukleinsäure enthält, in gramnegativen Bakterien repliziert wird, die Biomasse aufgeschlossen wird; eine Anionentauscherchromatographie durchgeführt wird; die löslichen Bestandteile an Hydroxylapatit chromatographiert werden und die genannte Nukleinsäure isoliert wird.

5. Verwendung einer replizierbaren Plasmid-DNA mit einem Gehalt von weniger als 0,1 % Protein sowie mit einem Gehalt von 0,01 bis 0,1 EU/mg an Endotoxinen, zur Herstellung eines Arzneimittels zur ex vivo und/oder in vivo Gentherapie.

**Claims**

1. Preparation of a plasmid DNA that can be replicated in gram-negative bacteria having a protein content of less than 0.1 % and an endotoxin content of 0.01 to 0.1 EU/mg DNA.

2. Preparation as claimed in claim 1, which is free from ethidium bromide, phenol, caesium chloride, MOPS buffer substance and detergents based on octylphenol-poly(ethylene glycol ether)$_n$.

3. Pharmaceutical composition containing a replicable plasmid DNA having a protein content of less than 0.1 % and an endotoxin content of 0.01 to 0.1 EU/mg DNA in a therapeutically effective amount and optionally pharmaceutical auxiliary substances, fillers or additives.

4. Process for producing a nucleic acid which has an endotoxin content of less than 1 EU/mg DNA, **characterized in that** a plasmid which contains the nucleic acid is replicated in gram-negative bacteria, the biomass is lysed; an anion exchange chromatography is carried out; the soluble components are chromatographed on hydroxylapatite and the said nucleic acid is isolated.

5. Use of a replicable plasmid DNA which has a protein content of less than 0.1 % and an endotoxin content of 0.01 to 0.1 EU/mg to produce a drug for *ex vivo* and/or *in vivo* gene therapy.

**Revendications**

1. Préparation d'un ADN plasmidique capable de réplication dans des bactéries à Gram négatif présentant une teneur inférieure à 0,1 % en protéine et une teneur de 0,01 à 0,1 UE/mg en ADN d'endotoxines.

2. Préparation selon la revendication 1, dépourvue de bromure d'éthidium, de phénol, de chlorure de césium, de substance tampon MOPS et de détergents à base d'octylphénolpoly(éther d'éthylèneglycol)$_n$.

3. Composition pharmaceutique, contenant un ADN plasmidique réplicable présentant une teneur inférieure à 0,1 % en protéine et une teneur de 0,01 à 0,1 UE/mg en ADN d'endotoxines dans une quantité thérapeutiquement efficace et éventuellement des excipients, agents de charge ou adjuvants pharmaceutiques.

4. Procédé de production d'un acide nucléique présentant une teneur inférieure à 1 UE/mg en ADN d'endotoxines, **caractérisé en ce qu'**un plasmide qui contient l'acide nucléique est répliqué dans des bactéries à Gram négatif, la biomasse est digérée ; une chromatographie échangeuse d'anions est réalisée ; les composants solubles d'hydroxylapatite sont chromatographiés et ledit acide nucléique est isolé.

5. Utilisation d'un ADN plasmidique réplicable présentant une teneur inférieure à 0,1 % en protéine et une teneur de 0,01 à 0,1 UE/mg en endotoxines, pour la production d'un médicament pour une thérapie génique ex *vivo* et/ou *in vivo.*

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0268946 B
- WO 9521177 A
- WO 9521179 A


**In der Beschreibung aufgeführte Nicht-Patentliteratur**

- **BIRNBOIM ; DOLY.** *Nucleic Acid Res.,* 1979, vol. 7, 1513-1523
- **GARGER et al.** *Biochem. Biophys. Res. Comm.,* 1983, vol. 117, 835-842
- QIAGEN® Plasmid Handbook. Qiagen Inc,
- **CHANDRA et al.** Mono-Q, Source-Q von Pharmacia, Macroprep-Q von BioRad, Poros-Q von Perspective Biosystems oder HyperD-Q von Biosepra. *Analyt. Biochem.,* 1992, vol. 203, 169-172
- **DION et al.** *J. Chrom.,* 1990, vol. 535, 127-147
- **MCCLUNG ; GONZALES.** *Analyt. Biochem.,* 1989, vol. 177, 378-382
- **RAYMOND et al.** *Analyt. Biochem.,* 1988, vol. 173, 125-133
- **VON COTTEN et al.** *Gene Therapy,* 1994, vol. 1, 239-246
- **FIEDLER.** Lexikon der Hilfsstoffe für Pharmazie und Kosmetik und angrenzende Gebiete. 1989, vol. 9
- **JOHNSON ; ILAN.** *Analyt. Biochem.,* 1983, vol. 132, 20-25
- Protein Purification Methods. Oxford University Press, 1989, 238-244
- **VON J. SAMBROOK et al.** Molecular Cloning: A Laboratory Manual. Cold Spring Harbor Laboratory Press, 1989
- Current Protocols in Molecular Biology. Wiley Interscience, 1991
- **BIRNBOIM ; DOLY.** *Nucleic Acids Research,* 1979, vol. 7, 1513-1523
- **BIRNBOIM et al.** *Nucl. Acids Res.,* 1979, vol. 7, 1513-1523
- *Meth. Enzymol.,* 1983, vol. 100, 243-255
- *Qiagen News,* Januar 1996, 3-5
- **BIRNBOIM, H.C. ; DOLY, J.** *Nucleic Acids Research,* 1979, vol. 7, 1513-1523
- **BIRNBOIM, H.C. et al.** *Meth. Enzymol.,* 1983, vol. 100, 243-255
- **CHANDRA et al.** *Analyt. Biochem.,* 1992, vol. 203, 169-172
- **COTTEN et al.** *Gene Therapy,* 1994, vol. 1, 239-246
- **DION et al.** *J. Chrom.,* 1990, vol. 535, 127-147
- **GARGER et al.** *Biochem. Biophys. Res. Comm.,* 1983, vol. 117, 835-842
- **JOHNSON ; ILAN.** *Analyt. Biochem.,* 1983, vol. 132, 20-25
- **MCCLUNG ; GONZALES.** *Analyt. Biochem.,* 1989, vol. 177, 378-382
- Protein Purification Methods. Oxford University Press, 1989, 238-244
- *QIAGEN NEWS,* Januar 1996, 3-5
- QIAGEN Plasmid Handbook. Qiagen Inc
- **RAYMOND et al.** *Analyt. Biochem.,* 1988, vol. 173, 125-133
- **SAMBROOK, J. et al.** Molecular Cloning: A Laboratory Manual. Cold Spring Harbor Laboratory Press, 1989